# EUROPÄISCHE PATENTSCHRIFT

(11) **EP 1 080 107 B1**
(45) Veröffentlichungstag und Bekanntmachung des Hinweises auf die Patenterteilung: **04.01.2012**
(21) Anmeldenummer: 99927793.2
(22) Anmeldetag: 28.05.1999
(51) Int. Cl.: C07K 14/03, C07K 16/08, C12N 15/38, A61K 38/16, A61K 39/245, G01N 33/68

(54) **NEUE PEPTIDE, DIE EPITOPE DES HUMANEN HERPESVIRUS 8 DARSTELLEN**
NOVEL PEPTIDES WHICH REPRESENT THE EPITOPES OF THE HUMAN HERPES VIRUS TYPE 8
NOUVEAUX PEPTIDES REPRESENTANT DES EPITOPES DU VIRUS 8 DE L'HERPES HUMAIN

(30) Priorität: 29.05.1998 DE 19824244
(43) Veröffentlichungstag der Anmeldung: 07.03.2001
(73) Patentinhaber: Biotrin Intellectual Properties Limited, Mount Merrion, County Dublin (IE)
(72) Erfinder: Schatz, Octavian, 85250 Altomünster (DE); Haas, Jürgen, 80689 München (DE)
(74) Vertreter: Vossius & Partner
(86) Internationale Anmeldenummer: PCT/EP1999/003719
(87) Internationale Veröffentlichungsnummer: WO 1999/062938

(56) Entgegenhaltungen:
- EP-A- 0 893 504
- EP-A- 0 950 666
- WO-A-96/15779
- WO-A-97/31932
- WO-A-98/04576
- WO-A-98/11132
- TEDESCHI E.A.: "Human serum antibodies to a major defined epitope of HHV8 small viral capsid antigen" J.INFECTIOUS DISEASES, Bd. 179, Nr. 4, April 1999 (1999-04), Seiten 1016-1020, XP000857350
- PAU CP ET AL: "Mapping and serodiagnostic application of a dominant epitope within the human herpesvirus 8 ORF 65-encoded protein" JOURNAL OF CLINICAL MICROBIOLOGY,US,WASHINGTON, DC, Bd. 36, Nr. 6, Seite 1574-1577-1577 XP002103027 ISSN: 0095-1137
- DAVIS DA ET AL: "Detection of serum antibodies to a Kaposi's sarcoma- associated herpesvirus-specific peptide" JOURNAL OF INFECTIOUS DISEASES,US,CHICAGO, IL, Bd. 175, Nr. 5, Seite 1071-1079-1079 XP002103028 ISSN: 0022-1899 in der Anmeldung erwähnt

### SEQUENZPROTOKOLL

(1) ALLGEMEINE INFORMATION:
   (i) ANMELDER:
      (A) NAME: Dr. Octavian SCHATZ
      (B) STRASSE: Steinbergstr. 30
      (C) ORT: Altomünster
      (D) BUNDESLAND: Bayern
      (E) LAND: Deutschland
      (F) POSTLEITZAHL: 85250

      (A) NAME: Dr. Juergen HAAS
      (B) STRASSE: Brunellenweg 39
      (C) ORT: München
      (D) BUNDESLAND: Bayern
      (E) LAND: Deutschland
      (F) POSTLEITZAHL: 80689
   (ii) ANMELDETITEL: Neue (Poly)peptide, die Epitope des humanen Herpesvirus 8 darstellen
   (iii) ANZAHL DER SEQUENZEN: 11
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 1:
   Met Tyr Glu Val Phe Thr Asp Phe Pro Val
   1               5                   10
(2) INFORMATION ZU SEQ ID NO: 2:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 2:
   Asp Pro Ala Tyr Thr Asn Asn Thr Glu Ala
   1               5                   10
(2) INFORMATION ZU SEQ ID NO: 3:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 3:
   Arg His Met Tyr Lys Pro Ile Ser Pro Gln
   1               5                   10
(2) INFORMATION ZU SEQ ID NO: 4:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 4:
   Met Ser Asn Phe Lys Val Arg Asp Pro Val
   1               5                   10
(2) INFORMATION ZU SEQ ID NO: 5:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 5:
   Ala Arg Lys Pro Pro Ser Gly Lys Lys Lys
   1               5                   10
(2) INFORMATION ZU SEQ ID NO: 6:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 6:
   Gln Glu Glu Gln Glu Leu Glu Glu Val Glu
   1               5                   10
(2) INFORMATION ZU SEQ ID NO: 7:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 7:
   Ala Ile Pro Pro Leu Val Cys Leu Leu Ala
   1               5                   10
(2) INFORMATION ZU SEQ ID NO: 8:
   (i) SEQUENZ CHARAKTERISTIKA:
      (A) LÄNGE: 12 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 8:
   Pro Thr Tyr Arg Ser His Leu Gly Phe Trp Gln Glu
   1               5                   10
(2) INFORMATION ZU SEQ ID NO: 9:
   (i) SEQUENZ CHARAKTERISTIKA:
      (A) LÄNGE: 14 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 9:
   Ser Gly Ser Gly Ile Trp Gly Cys Ser Gly Lys Leu Ile Cys
   1               5                   10
(2) INFORMATION ZU SEQ ID NO: 10:
   (i) SEQUENZ CHARAKTERISTIKA:
      (A) LÄNGE: 14 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 10:
   Ser Gly Ser Gly Gln Glu Pro Pro Ala Pro Gln Ala Pro Ile
   1               5                   10
(2) INFORMATION ZU SEQ ID NO: 11:
   (i) SEQUENZ CHARAKTERISTIKA:
      (A) LÄNGE: 14 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 11:
   Ser Gly Ser Gly Pro Ala Leu Thr Ala Val Glu Thr Gly Ala
   1               5                   10

## Beschreibung

Die vorliegende Erfindung betrifft die Verwendung von Peptiden, die von anti-HHV8 Antikörpern HHV8-infizierter Patienten erkannt werden, wobei diese Peptide nicht natürlich vorkommende HHV8-Proteine sind in Verfahren, um sehr sensitiv und spezifisch anti-HHV8 Antikörper nachzuweisen. Außerdem betrifft die vorliegende Erfindung einen diagnostischen Kit, der die erfindungsgemäßen Peptide umfaßt und zum Nachweis von anti-HHV8 Antikörpern bzw. zur Diagnose einer HHVB-Infektion eingesetzt werden kann.

Das humane Herpesvirus 8 (HHV8) ist das mutmaßliche ätiologische Agens des Kaposi Sarkoms und bestimmter B-Zell Lymphome. Das Kaposi Sarkom (KS) ist der weitaus häufigste Tumor bei AIDS Patienten. Man schätzt, daß 20 bis 30% aller Patienten im Laufe ihrer HIV Infektion an KS erkranken. In den U.S.A. ist die Inzidenz damit 20.000 x höher als in der Gesamtpopulation (1). In manchen geografischen Regionen, wie z.B. in den Ländern des Mittelmeerraums oder Afrika liegt die Gesamtinzidenz deutlich höher.
AIDS-assoziiertes KS ist durch einen aggressiven Verlauf und hohe Mortalität gekennzeichnet, während der Krankheitsverlauf beim klassischen KS meist relativ indolent und langsam progredient ist. Weitere Formen dieser Erkrankung sind das sogenannte "endemische" KS, das vor allem bei Kindern und jugendlichen Erwachsenen in Afrika südlich der Sahara auftritt sowie das iatrogene KS bei immunsupprimierten Transplantationsempfängern.
KS wurde erstmals vor mehr als 100 Jahren als ein relativ seltener Tumor beschrieben, der vor allem bei älteren Männern mediterranen oder osteuropäischen Ursprungs auftrat (2). Äußerlich manifestiert sich KS durch scharf umgrenzte, noduläre Läsionen der Haut meist im Bereich der Extremitäten; die Läsionen werden aber auch an den Schleimhäuten und Viszera beobachtet. Charakteristisches histologisches Merkmal dieser Läsionen ist die Präsenz länglicher, spindelförmiger Zellen vermutlich endothelialen Ursprungs. Darüber hinaus findet man eine Anzahl weiterer Zelltypen wie Monozyten, Fibroblasten, neovaskuläre Strukturen und extravasale Erythrozyten. Im Tumorgewebe ist die Produktion von VEGF (vascular endothelial growth factor) signifikant erhöht; dies hat eine andauernde Angioneogenese und somit eine extreme Vaskularisierung zur Folge.
Epidemiologische Untersuchungen ergaben schon vor einigen Jahren Hinweise auf die Beteiligung eines infektiösen Agens (1, 3). Mittels einer neuen PCR Technik wurden 1994 in Biopsiematerial von KS Läsionen Sequenzen eines bis dato noch unbekannten humanen γ2-Herpesvirus gefunden (4). Das dazugehörige Virus wurde KSHV (Kaposi Sarkom assoziiertes Herpesvirus) oder HHV8 (Humanes Herpesvirus 8) genannt. Das 140 kb große Genom ist vor kurzem kloniert und sequenziert worden (5).
Aufgrund einer ganzen Reihe von Arbeiten besteht heute kein Zweifel mehr daran, daß das Auftreten eines Kaposi Sarkoms gleich welcher Form zu praktisch 100% mit der Präsenz von HHV8 in den Läsionen korreliert ist (6-11). Der PCR Nachweis von HHV8 DNA ist zudem ein prognostisches Indiz für die spätere Entwicklung eines Kaposi Sarkoms (12). Unsere eigenen Arbeiten zeigen, daß eine Serokonversion im Mittel bereits 2 Jahre vor dem Auftreten eines Kaposi Sarkoms nachweisbar ist.
Wahrscheinlich ist HHV8 auch an der Pathogenese bestimmter Lymphome wie etwa Castleman's Disease oder PEL (Primary Effusion Lymphoma) beteiligt (13, 14). Darüber hinaus gibt es Hinweise, daß HHV8 auch bei interstitieller Pneumonie und Enzephalitis eine Rolle spielen könnte (15). Schließlich wurde in einer kürzlich publizierten Arbeit eine Korrelation zwischen dem Auftreten von HHV8 und multiplen Myelomen postuliert (16). Obwohl verschiedene experimentelle Befunde (17-22) die Hypothese stützen, daß HHV8 in die Pathogenese dieser Krankheiten involviert sein könnte, ist ein kausaler Zusammenhang bislang noch nicht endgültig bewiesen. In manchen Regionen (z.B. in Zentralafrika) scheint HHV8 nämlich auch in der Gesamtbevölkerung relativ weit verbreitet zu sein (6, 8, 23, 24). Trotz einer erhöhten Inzidenz des Kaposi Sarkoms in diesen Ländern scheint das Vorliegen einer Infektion mit HHV8 alleine ein zwar notwendiges, aber nicht hinreichendes Kriterium für die Entwicklung der Krankheit zu sein.
Anhand der gefundenen viralen DNA Fragmente wurden verschiedene diagnostische Verfahren entwickelt, die sowohl einen direkten als auch einen indirekten Nachweis von HHV8 erlauben. Durch einen relativ einfachen Test, der Polymerase-Kettenreaktion (PCR), können selbst sehr geringe Mengen viraler DNA in Blut- oder Gewebeproben bestimmt werden (25-28). Allerdings ist auch dieser Test für manche medizinisch relevanten Untersuchungen nicht sensitiv genug.
Künstlich immunsupprimierte Organtransplantierte entwickeln ebenfalls überdurchschnittlich häufig ein Kaposi Sarkom (bei Nierentransplantationen bis zu 5%), wobei die Zahl der Fälle stark je nach geografischer Herkunft variiert. So ist das KS in den Ländern des Nahen und Mittleren Ostens der häufigste Posttransplantationstumor und liegt weltweit an 3. Stelle. In den Industrieländern (Europa, Japan, U.S.A.) haben insgesamt 1-1,5 Millionen Menschen ein erhöhtes Risiko, an KS zu erkranken. Für diese Personen bestimmte Blutpräparate sollten daher sicherheitshalber unbedingt auf HHV8 Antikörper (als Indiz für eine mögliche Verseuchung mit HHV8) getestet werden, um deren Infektionsrisiko so gering wie möglich zu halten. Im erweiterten Sinn trifft dies auch andere Personenkreise, bei denen eine mehr oder weniger stark ausgeprägte Beeinträchtigung des Immunsystems vorliegt, beispielsweise Patienten, die eine Chemotherapie erhalten, Dialysepatienten, ältere Patienten, Neonate etc.
Da KS hauptsächlich Personen mit einer Immunschwäche gleich welcher Art betrifft und nur mit mäßigem Erfolg chemotherapeutisch, chirurgisch oder mit ionisierenden Strahlen behandelbar ist (es gibt derzeit weder eine kausale Therapie noch eine endgültige Heilung für diese Krankheit), muß man davon ausgehen, daß immundefiziente Personen durch die Verabreichung HHV8-kontaminierter Blutpräparate einem besonderen Risiko ausgesetzt sind, ein Kaposi Sarkom zu entwickeln.
In der Routinediagnostik kommen meist sogenannte ELISA (enzyme linked immunosorbent assay)-Testverfahren zum Einsatz, da sie sehr billig sind und sich auch für hohe Probenaufkommen eignen. Der Nachweis von HHVB-spezifischen Antikörpern im Serum ist ein Indiz für einen (früheren) Kontakt mit dem Antigen. Dieser kann unter Umständen auch dann noch positiv ausfallen, wenn die Menge an viraler DNA unterhalt der Nachweisgrenze der PCR liegt. Die Anwesenheit HHV8 spezifischer Antikörper kann außer mit dem ELISA (31-33) auch mittels indirektem Fluoreszenzassay (IFA) (24) und Western-Blot (29, 30) untersucht werden.
WO 98/11132 beschreibt ein 85 Aminosäuren langes Polypeptid des carboxyterminalen Endes von KSHV orf 65 zur Diagnose von Kaposi's Sarkom oder HHV8. Auch WO 97/31932 beschreibt ein 60 Aminosäure langes Protein aus HHV8 zur Diagnose von Kaposi's Sarkom oder HHV8.
EP 0950666 beschreibt eine antigenen Peptidsequenz aus dem ersten orf von T0.7 sowie die Herstellung monoklonaler Antikörper gegen diese Sequenz. Die Verwendung des Peptids sowie der Antikörper zur Diagnose und Therapie von Kaposi's Sarkom oder HHV8 wird beschrieben.
Allerdings sind die bislang beschriebenen Verfahren entweder sehr zeitaufwendig (IFA bzw. Westem-Blot) oder nicht sensitiv genug (PCR aus Blutproben, ELISA mit herkömmlichen Antigenen).

Das der vorliegende Erfindung zugrundeliegende technische Problem war somit, Reagenzien zur Verfügung zu stellen, deren Einsatz einen sehr sensitiven und spezifischen Nachweis von anti-HHV8 Antikörpern ermöglicht.

Dieses technische Problem wird durch die Bereitstellung der in den Patentansprüchen gekennzeichneten Ausführungsformen gelöst.

Dementsprechend betrifft die vorliegende Erfindung ein Verfahren zum Nachweis von anti-HHV8 Antikörpern durch ein Peptid, das von anti-HHV8 Antikörpern HHVB-infizierter Patienten erkannt wird und dadurch gekennzeichnet ist, daß es
(a) eine der in SEQ ID NR.: 1 bis 4, 6, und 8 gekennzeichneten Aminosäuresequenzen umfaßt;
(b) aus einer der in SEQ ID NR.: 1 bis 4, 6 und 8 gekennzeichneten Aminosäuresequenzen besteht;
wobei das (Poly)peptid kein natürlich vorkommendes HHV8-Protein ist; wie in den Patentansprüchen charakterisiert.

Natürlich vorkommende HHV8-Proteine im Sinne dieser Erfindung sind solche HHV8-Proteine, die die vollständige Aminosäuresequenz aufweisen und nicht degradiert sind. Üblicherweise oder vorzugsweise wird das Peptid von anti-HHV8 Antikörpern in Körperflüssigkeiten von HHV8-infizierten Patienten erkannt. Methoden zur Bestimmung der antigenen Eigenschaft des efindungsgemäßen Peptids mittels anti-HHV8 Antikörper sowie Methoden zu deren Isolierung, vorzugsweise aus Körperflüssigkeiten HHVB-infizierter Patienten, sind dem Fachmann bekannt und umfassen beispielsweise den ELISA.

Der Begriff "Körperflüssigkeiten" umfaßt im Sinne der vorliegenden Erfindung z.B. Blut, Serum, Lymphe, Gewebeflüssigkeit und Gewebeextrakte z.B. aus Mucosae respiratorischen, urogenitalen oder gastrointestinalen Ursprungs. Desweiteren umfaßt dieser Begriff Körperflüssigkeiten, die derart behandelt bzw. vorbereitet wurden, daß sie z.B. für den Einsatz in den oben erwähnten Methoden geeignet sind. Beispiele hierfür sind in vitro verdünnte Seren, mit Konservierungsstoffen (wie z.B. vor Kälte schützende Substanzen) versetzte Körperflüssigkeiten oder mit gerinnungshemmenden Stoffen (z.B. Heparin) behandelte Körperflüssigkeiten.

Erfindungsgemäße Peptide, die eine der in SEQ ID NR.: 1 bis 4, 6 und 8 gekennzeichneten Aminosäuresequenzen umfassen, können beispielsweise von einer HHV8-Sequenz und/oder einer anderen Sequenz flankiert sein, die nicht von HHV8 abstammt. Wie oben bereits erwähnt, ist das erfindungsgemäße Peptid jedoch kein natürlich vorkommendes HHVB-Protein, d.h. das erfindungsgemäße Peptid stellt kein volle-Länge-HHV8-Protein dar, wie es von HHV8-Genomen codiert wird.

Im sinne des vorliegenden Erfindung stellen die erfindungsgemäßen Peptide lineare Epitope verschiedener kodierender Regionen der HHV8-Genoms dar und besitzen eine Länge von höchstens 15 Aminosäuren

Der Begriff "Substitution" umfaßt im Sinne der vorliegenden Erfindung sowohl konservative als auch nicht-konservative Aminosäureaustausche. Unter konservative Aminosäureaustausche fallen solche, bei denen beispielsweise eine neutrale, hydrophobe, eine neutrale, polare, eine basische oder eine saure Aminosäure durch eine Aminosäure derselben Klasse ersetzt wird. Dem Fachmann sind die verschiedenen Klassen von Aminosäuren, deren Klassenzugehörigkeit durch die spezifischen Seitengruppen bestimmt ist, wohl bekannt. Wesentlich dabei ist, daß die dreidimensionale Struktur der Peptide nicht derart verändert wird, daß sie von anti-HHV8 Antikörpern nicht mehr erkannt werden.

Die erfindungsgemäßen Peptide wurden in einem Screening von ca. 3000 Peptiden identifiziert, das nach folgenden Kriterien durchgeführt wurde:
(a) Minimale Homologie mit Peptiden bekannter Proteine, vor allem mit entsprechenden Epitopen verwandter Herpesviren wie z.B. EBV und CMV; und
(b) Maximale Antigenität (laut Computervorhersage).

Eine Vorselektion möglicherweise immundominanter, HHV8-spezifischer Peptide wurde durch den Einsatz einer vorteilhaften Kombination verschiedener Computerprogramme (BLASTP, ANTIGENIC und BLASTALIGN) der Genetics Computer Group (GCG), Wisconsin durchgeführt.
Alle Peptide, die in dieser Vorselektion ausgewählt wurden (292 Peptide), wurde dann einzeln mit einem Serumpool von KS Patienten und verschiedenen Kontrollpools von gesunden Probanden analysiert.
Die-aus diesem Initialscreen hervorgegangen Peptide wurden daraufhin an mehreren hundert Seren von KS Patienten und Kontrollen getestet, wobei spezielles Augenmerk darauf gerichtet wurde, ob Kreuzreaktionen mit EBV-, CMV- oder HSV spezifischen Antikörpern auftraten.
Durch diese mehrfachen Tests wurden die erfindungsgemäßen Peptide, die das gewünschte Reaktionsprofil aufwiesen, identifiziert.

Die erfindungsgemäßen Peptide umfassen auch durch Peptidomimetics hergestellte Verbindungen, vorzugsweise Peptide, die den vorgenannten Peptiden in den immunologischen/diagnostischen Eigenschaften entsprechen. Dem Fachmann ist bekannt, wie solche Moleküle z.B. unter Verwendung von L- und D-Aminosäuren entworfen und hergestellt werden (z.B. Banerjee, Biopolymers 39 (1996), 769-777).

Vorzugsweise betrifft die vorliegende Erfindung die Verwendung eines Peptidmix, welcher aus einer Kombination erfindungsgemäßer Peptide besteht.

In einer bevorzugten Ausführungsform umfaßt der Peptidmix die Peptide mit den in SEQ ID NR.: 1, 3, 4 und 8 gekennzeichneten Aminosäuresequenzen.

In einer besonders bevorzugten Ausführungsform enthält der erfindungsgemäße Peptidmix diese Peptide in einem molaren Verhältnis von 1:1:1:1.

Unter Verwendung des erfindungsgemäßen Peptidmixes können anti-HHV8 Antikörper in besonders vorteilhafter Weise nachgewiesen werden, wobei dieser Nachweis überraschenderweise sowohl die Empfindlichkeit als auch die Spezifität herkömmlicher Nachweisverfahren weit übertrifft (siehe unten).

Auch beschrieben hierin ist ein Peptid, das eine Aminosäuresequenz umfaßt, die von einer der in (a) gekennzeichneten Aminosäuresequenzen durch eine oder mehrere Substitutionen Deletionen und/oder Insertionen abweicht vorzugsweise ist dieses Peptid eine natürlich vorkommende Sequenzvariante.

Solche Peptide, die Sequenzvarianten der entsprechenden Positionen im Genom von HHV8 (5) darstellen, können die Sensitivität des Nachweises noch weiter erhöhen. Die Spezifität bleibt dabei erhalten, da die Auswahl der Peptide, unter anderem nach minimaler Homologie zu bekannten (viralen) Proteinen, hochkonservierte Bereiche von vornherein ausschließt.

Des weiteren ist hierin ein Polymer beschrieben, das mindestens zwei gleiche oder verschiedene erfindungsgemäße Peptide umfaßt.

Dabei umfassen die Polymere Homopolymere, bestehend aus mehreren Exemplaren eines einzigen erfindungsgemäßen Peptids, als auch Heteropolymere aus einer beliebigen Kombination der erfindungsgemäßen Peptide, wobei solche Heteropolymere auch mehrere Exemplare eines erfindungsgemäßen Peptids enthalten können.

Auch beschrieben ist, dass das erfindungsgemäße Polymer durch eine unverzweigte Kette von polymerisierten erfindungsgemäßen Peptiden gekennzeichnet, ist.

Das weiteren ist beschrieben, dass das erfindungsgemäße Polymer durch verzweigte Ketten von polymerisierten erfindungsgemäßen Peptiden gekennzeichnet ist.

Polymere mit verzweigten Ketten von polymerisierten Peptiden werden z.B. durch Verknüpfung der Peptide über eine Aminosäure oder ein Aminosäure-Analogon hergestellt, die/das zwei Aminogruppen und eine Carboxylgruppe besitzen, wobei jede Gruppe fähig ist, eine Peptidbindung zu bilden. Dem Fachmann sind solche Synthese-Verfahren bekannt.

Auch beschrieben sind Polymere, die die Peptide mit den in SEQ ID NR.: 1, 3, 4 und 8 gekennzeichneten Aminosäuresequenzen umfassen.

In einer weiteren bevorzugten Ausführungsform ist das erfindungsgemäße Peptid oder das hier beschriebene Polymer chemisch synthetisiert.

Synthese-Verfahren zur chemischen Herstellung von Peptiden mittels Peptid-Synthesizer sind dem Fachmann bekannt.

Auch Gemische bestehend aus den erfindungsgemäßen Polymeren sind hier beschrieben.

Des weiteren ist ein Fusionprotein beschrieben, das ein erfindungsgemäßes Peptide oder ein hier beschriebenes Polymer umfaßt.

Auch beschreiben ist ein Polynukleotid, das ein erfindungsgemäßes Peptid, Polymer oder Fusionsprotein codiert.

In einer bevorzugten Ausführungsform ist das Polynukleotid DNA oder RNA.

Des weiteren ist ein Vektor beschrieben der ein oben beschriebenes Polynukleotid umfaßt. In einer bevorzugten Ausführungsform ist der beschriebene Vektor ein Expressionsvektor.

Der Begriff "Expressionsvektor" umfaßt im Sinne der vorliegenden Erfindung sowohl prokaryontische als auch eukaryontische Expressionsvektoren. Die notwendigen regulatorischen Elemente zur Expression eines Peptids sind dem Fachmann wohl bekannt und können den Expressionsbedingungen entsprechend ausgewählt werden. Der Begriff "Expression" kann dabei Transkription als auch Transkription und Translation bedeuten. Regulatorische Elemente umfassen dabei insbesondere Promotoren. Für die Expression eines Polynucleotids in prokaryontischen Zellen stehen eine Reihe von Promotoren zur Verfügung, z.B. der E. coli lac- oder trp-Promotor, der P_{R}- oder P_{L}-Promotor des Lambda-Phagen, lacI, lacZ. T3, T7, gpt, etc. Eukaryontische Promotoren sind beispielsweise der CMV immediate early-Promotor, der HSV-Promotor, der Thymidinkinase-Promotor, der SV40-Promotor, der LTRs von Retroviren und der Maus Metallothioninl-Promotor. Es ist bereits eine Vielzahl von Expressionsvektoren für die Expression in prokaryontischen oder eukaryontischen Zellen beschrieben, z.B. für Eukaryonten pKK223-3 (Pharmacia Fine Chemicals, Uppsala, Schweden) oder GEM1 (Promega Biotec, Madison, WI, USA), pSV2CAT, pOG44 und für Prokaryonten pQE70, pQE60, pBluescript SK, etc. Neben Promotoren können erfindungsgemäße Vektoren auch Elemente zur weiteren Steigerung der Transkription enthalten, wie z.B. sogenannte Transkriptions-Enhancer. Beispiele dafür sind der SV40-Enhancer, der Polyoma-Enhancer, der Cytomegalovirus early Promoter-Enhancer und Adenovirus-Enhancer.

Des weiteren ist eine Wirtszelle beschrieben die einen oben beschriebenen Vektor enthält.

Unter den Begriff "Wirtszelle" fallen sowohl prokaryontische als auch eukaryontische Wirtszellen. Bevorzugte prokaryontische Wirtszellen umfassen z.B. E. coli-Zellen, Streptomyces-, Bacillus- oder Salmonella-Zellen. Bevorzugte eukaryontische Wirtszellen umfassen Pilzzellen, z.B. Hefezellen, insbesondere Saccharomyces cerevisae-Zellen, Insektenzellen wie z.B. Drosophila- oder SF9-Zellen, tierische Zellen wie z.B. CHO- oder COS-Zellen, Pflanzenzellen oder Säugerzellen. Auch beschrieben ist ein Verfahren zur Herstellung eines erfindungsgemäßen (Peptids, Polymers oder Fusionsproteins, das folgende Schritte umfaßt:
(a) Züchtung der beschriebenen Wirtszelle unter Bedingungen, die die Expression des Peptids, des Polymers oder des Fusionsproteins bewirken; oder
(b) in vitro Transkription und/oder Translation des beschriebenen Polynukleotids;
und Gewinnung des in (a) oder (b) hergestellten Peptids, Polymers oder Fusionsproteins.

Die Bedingungen, die eine Expression eines Peptids bewirken sind dem Fachmann bekannt und können entsprechend der verwendeten Wirtszelle und des eingesetzten Expressionsvektors gewählt werden. Entsprechend gestaltet sich auch die Gewinnung des Expressionsproduktes. Wird z.B. ein Expressionsvektor eingesetzt, der zur-Sekretion des synthetisierten Peptids führt, wird das Peptid aus dem Kulturüberstand gewonnen. Findet die Expression intrazellulär statt, wird das Expressionsprodukt aus der Wirtszelle isoliert.
Methoden zur in vitro Transkription und/oder Translation sind dem Fachmann ebenfalls wohl bekannt. Beispielsweise lassen sich solche Verfahren mittels kommerzieller Kits entsprechend den Instruktionen des Herstellers durchführen.

Vorzugsweise werden bei der Gewinnung des erfindungsgemäßen Peptids, Polymers oder Fusionsproteins zusätzliche, dem Fachmann bekannte Reinigungsschritte, wie z.B. säulenchromatographische Verfahren, durchgeführt, um Verunreinigungen (z.B. zelluläre Proteine, Nucleinsäuren, Komponenten des in vitro Transkriptions-/Translations-Systems) weitestgehend zu entfernen.

Auch beschrieben hier ist ein Konjugat, das ein erfindungsgemäßes Peptid. Polymer oder Fusionsprotein, oder ein Peptid, Polymer oder Fusionsprotein, hergestellt durch das beschriebene Verfahren, umfaßt.

Des weiteren beschrieben ist eine Zusammensetzung, die mindestens ein erfindungsgemäßes Peptid, und/oder ein beschriebenes Polymer, und/oder ein beschriebenes Fusionsprotein, und/oder ein Peptid, Polymer oder Fusionsprotein hergestellt durch das beschriebene Verfahren und/oder ein beschriebenes konjugat umfaßt und gegebenenfalls einen pharmazeutisch verträglichen Träger und/oder ein pharmazeutisch verträgliches Verdünnungsmittel.

In einer bevorzugten Ausführungsform ist die Zusammensetzung ein Arzneimittel.

In einer besonders bevorzugten Ausführungsform ist das Arzneimittel ein Impfstoff. In dieser Ausführungsform sind die Peptide, einzeln oder in Kombination, vorzugsweise an einen Carrier gekoppelt. Beispiele für geeignete Carrier sind Polystyrolkugelchen, Streptavidin, BSA oder KLH.

Beispiele für geeignete pharmazeutisch verträgliche Träger und/oder Verdünnungsmittel sind dem Fachmann bekannt und umfassen z.B. Phosphatgepufferte Kochsalzlösungen, Wasser, Emulsionen, wie z.B. Öl/Wasser-Emulsionen, verschiedene Arten von Netzmittel oder Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,001 und 1000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 1 µg- und 10 mg-Einheiten pro Tag befinden. Wird die Zusammensetzung intravenös verabreicht, was nicht bevorzugt empfohlen wird, um die Gefahr einer anaphylaktischen Reaktionen zu minimieren, sollte sich die Dosis in einem Bereich zwischen 1 µg- und 10 mg-Einheiten pro Kilogramm Körpergewicht pro Minute befinden.
Die Zusammensetzung der Erfindung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäße Zusammensetzung kann außerdem Konservierungsmittel und andere Zusätze umfassen, wie z.B. antimikrobielle Verbindungen, Antioxidantien, Komplexbildner und inerte Gase. Desweiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein.
Somit betrifft die vorliegende Erfindung ein Verfahren zum Nachweis von anti-HHV8 Antikörpern, das folgende Schritte umfaßt:
(a) inkontaktbringen einer biologischen Probe mit mindestens einem erfindungsgemäßen Peptid,
   unter Bedingungen, die die Bindung von Antikörpern erlauben; und
(b) Nachweis der in Schritt (a) gebunden Antikörper, wie in den Patentansprüchen charakterisiert.
Neben dem Nachweis von anti-HHV8 Antikörpern eignet sich das erfindungsgemäße Verfahren auch als prognostisches Indiz für die mögliche Entwicklung eines Kaposi Sarkoms bei HIV Infizierten bzw. Personen mit anderweitigen natürlichen oder iatrogenen Immundefekten.

In einer bevorzugten Ausführungsform wird bei diesem Verfahren ein Gemisch der Peptide, die die in SEQ ID NR.:1, 3, 4 und 8 gekennzeichneten Aminosäuresequenzen umfassen, verwendet.

Die wesentlichen Vorteile des erfindungsgemäßen Verfahrens gegenüber bisher beschriebenen Verfahren (24, 29-34) sind einerseits die sehr hohe Sensitivität (>96% der Seren von KS Patienten reagieren positiv, während die meisten in der Literatur beschriebenen Verfahren lediglich eine Sensitivität von 35-85% erreichen) und andererseits eine sehr gute Spezifität und Reproduzierbarkeit (bei Verwendung der ELISA-Technik zeigen Doppelbestimmungen als Resultat der gewählten Beschichtungstechnik eine durchschnittliche Abweichung von weniger als 5%). Insbesondere wird durch den Einsatz der erfindungsgemäßen Kombination verschiedener Peptide aus verschiedenen offenen Leserastern des viralen Genoms statt einzelner rekombinanter Antigene wie ORF65 (32) oder dem Minor Capsid Protein (33) ein größeres Spektrum von HHV8 spezifischen Antikörpern erkannt. Dies ist vor allem wichtig bei Seren von Patienten, bei denen bereits vor dem Erstkontakt mit HHV8 eine ausgeprägte Immundefizienz vorlag, da in diesen Fällen die Antikörpertiter oft sehr niedrig sind. Andere Peptid-basierte Immunoassays auf der Basis eines einzigen Peptids (31) erreichen eine weitaus geringere Sensitivität. Es ist zudem von anderen Herpesviren bekannt, daß die Verwendung eines einzelnen Epitops nicht ausreicht, um eine diagnostisch verwertbare Erkennungsquote von mindestens 90% aller positiven Seren zu erreichen.

In einer besonders bevorzugten Ausführungsform dieses Verfahrens sind die erfindungsgemäßen Peptide chemisch synthetisiert.

Durch die Verwendung chemisch synthetisierter Peptide anstelle rekombinanter Antigene, wie sie in anderen beschriebenen Verfahren benützt wurden, die auf der Verwendung prokaryontischer Expressionssystemen bzw. ungereinigter Viruslysate aus infizierten Zellinien beruhen, ist somit eine Reaktion von nichtspezifischen Antikörpern (z.B. gegen *E. coli* Proteine aber vor allem auch gegen Proteine anderer humaner Herpesviren wie beispielsweise EBV oder CMV) praktisch ausgeschlossen. In den bisher beschriebenen Verfahren müssen hohe Ansprüche an die Aufreinigung des Antigens gestellt werden, da selbst geringste Mengen an Verunreinigungen mit z.B. *E. coli* Proteinen bei vielen Proben zu falschpositiven Resultaten führen können. Vorteilhafterweise erübrigen sich bei dem erfindungsgemäßen Verfahren zeit- und damit kostenaufwendige Aufreinigungsschritte wie z.B. vorherige Adsorption der Testseren (z.B. an *E*. *coli* Lysat).

Vorzugsweise werden die erfindungsgemäßen Peptide, für den Einsatz in dem erfindungsgemäßen Verfahren biotinyliert. Die biotinylierten Komponenten werden dann über die hochspezifische Biotin-Streptavidin Interaktion an eine Streptavidinbeschichtete feste Phase gebunden. Dieses Kopplungsverfahren gewährleistet auch bei Gemischen von erfindungsgemäßen Peptiden unterschiedlicher Ladung bzw. Hydrophilie eine gleichbleibende Beschichtung.

In einer weiteren besonders bevorzugten Ausführungsform dieses Verfahrens umfaßt das Gemisch diese Peptide in einem molaren Verhältnis von 1:1:1:1.

In einer weiteren bevorzugten Ausführungsform ist das erfindungsgemäße Verfahren ein Enzym-gebundener Immunoabsorptionsassay (ELISA), ein Enzymimmunodotassay, ein Immunobead-Assay, ein passiver Hämagglutinations-Assay (PHA) oder ein Peptid-Antikörper-Peptid Sandwich-Assay.

In einer weiteren bevorzugten Ausführungsform des erfindungsgemäßen Verfahrens ist die biologische Probe eine behandelte oder unbehandelte Form von Blut, Serum, Gewebeextrakt, Gewebeflüssigkeit, Zellkulturüberstand, oder Zellysat. Bezüglich des Begriffs "behandelte Form" wird auf die entsprechende oben erwähnte Definition des Begriffs "Körperflüssigkeiten" verwiesen, die die Möglichkeiten der Behandlung bzw. Vorbereitung betreffend auch in diesem Zusammenhang anzuwenden ist.
In einer weiteren bevorzugten Ausführungsform umfaßt das erfindungsgemäße Verfahren den weiteren Schritt:
(c) Nachweis von unspezifischen Bindungen, die von in der biologischen Probe zusätzlich vorhandenen Antikörpern in Schritt (a) verursacht werden.

Im Falle eines ELISAs können zusätzlich vorhandene Antikörper z.B. antigenunabhängig bzw. unspezifisch an die Oberfläche der Vertiefungen einer Mikrotiterplatte binden und ein falsch-positives Signal bewirken. Falsch-positive Ergebnisse aufgrund unspezifischer Bindung an die Matrix werden durch individuelle Blankwerte sicher erkannt und eliminiert. Dies ist wichtig für den Einsatz im Screening von Blutbanken, um unnötiges Entfernen von Donorblut aufgrund falsch-positiver Ergebnisse für den HHV8 Nachweis zu vermeiden.

Die vorliegende Erfindung betrifft weiterhin einen Kit, enthaltend:
(a) mindestens ein erfindungsgemäßes Peptid wobei die Komponenten getrennt voneinander oder in Form eines Gemisches enthalten sind; und gegebenenfalls
(b) eine feste Phase, an die mindestens ein erfindungsgemäßes Peptid, gebunden werden kann;
(c) ein Probenverdünnungsmittel; und/oder
(d) eine Negativkontrolle; und/oder
(e) eine Positivkontrolle; und/oder
(f) ein Reportermolekül.

Der Begriff "Reportermolekül" umfaßt im Sinne der vorliegenden Erfindung sowohl Enzyme wie z.B. Peroxidase oder alkalische Phosphatase (in diesen Fällen kann das verwendete Detektionssystem z.B. auf einer kolorimetrischen Reaktion beruhen, etwa der Umsetzung von ABTS durch eine Peroxidase), andere Enzyme, Radioisotope, Fluorophore, bioluminiszierende Moleküle, chemiluminiszierende Moleküle oder dergleichen als auch deren Konjugate z.B. mit einem Zweitantikörper, der z.B. menschliche IgG- bzw. IgM-Antikörper erkennt.

Der erfindungsgemäße Kit kann zum Nachweis von anti-HHV8 Antikörpern und zur Diagnose von HHV8 Infektionen von Säugern vorzugsweise in Körperflüssigkeiten wie, z.B. Blut, Serum, Gewebeextrakten, Gewebeflüssigkeiten, aber auch *in vitro* Zellkulturüberständen und Zellysaten als Testreagentien in einem Immunoassay verwendet werden. Hierfür kommt jeder geeignete Immunoassay in Frage, insbesondere ein Enzym-gebundener Immunoabsorptionsassay (ELISA), ein Enzymimmunodotassay, ein Immunobead-Assay, ein passiver Hämagglutininations-Assay (PHA), ein Peptid-Antikörper-Peptid Sandwich Assay oder andere dem Fachmann bekannte Methoden.

In einer bevorzugten Ausführungsform enthält der Kit ein Gemisch der Peptide, die die in SEQ ID NR.: 1, 3, 4 und 8 gekennzeichneten Aminosäuresequenzen umfassen oder darstellen.

In einer weiteren Ausführungsform betrifft die vorliegende Erfindung die Verwendung mindestens eines erfindungsgemäßen Peptids zur Herstellung eines Arzneimittels zur Behandlung von HHVB-assoziierten Erkrankungen, wobei die HHV8-assoziierten Erkrankungen Kaposi Sarkom, Castleman's Disease, Primary Effusion Lymphoma, interstitieller Pneumonie, Enzephalitis und multiple Myelome sind.

In einer weiteren Ausführungsform betrifft die vorliegende Erfindung die Verwendung mindestens eines erfindungsgemäßen Peptids zur Herstellung von Antikörpern.
Die Herstellung von Antikörpern ist dem Fachmann bekannt und umfaßt beispielsweise die Immunisierung eines Tieres mit einem Antigen, das gegebenenfalls an einen Carrier gekoppelt ist und/oder in Kombination mit anderen immunstimulierenden Substanzen verabreicht wird (siehe z.B. Harlow und Lane, "Antibodies, a laboratory manual", Cold Spring Harbor Laboratory Press, Cold Spring Harbor, 1988). Andere bekannte Methoden umfassen das Screening von Antikörperbibliotheken mittels "phage display"-Technologie und die rekombinante Herstellung des gewünschten Antikörpers.

In einer weiteren Ausführungsform betrifft die vorliegenden Erfindung die Verwendung mindestens eines erfindungsgemäßen Peptids zum Nachweis von anti-HHV8 Antikörpern.

### Figur-Legenden:

**Figur 1****:**
   Die Serumaktivität gegen den bevorzugten Peptidmix wurde bei HIV⁺/KS⁺ Patienten ermittelt. Die Säulen zeigen den Mittelwert sowie die Standardabweichung für (i) positive Seren, (ii) Seren von Patienten mit einer CD4-Zellzahl von weniger als 50/µl, (iii) negative Seren. Die durchschnittliche Serumaktivität ist bei Patienten in fortgeschrittenen Krankheitsstadien deutlich geringer als für das Gesamtkollektiv.
**Figur 2****:**
   Serumaktivität und CD4-Zellzahl im Verlauf. Serumproben eines HIV⁺/KS⁺ Patienten wurden retrospektiv im HHV8 ELISA getestet. Bereits 14 Monate vor der Diagnose eines Kaposi Sarkoms sind HHV8-spezifische Antikörper im Blut des Patienten nachzuweisen. Die zusätzlich aufgetragenen CD4-Zellzahlen sind ein Maß für die relative Immundefizienz des Patienten.
**Figur 3****:**
   Schematische Darstellung der Entwicklung des HHV8 ELISA-Tests.
   Wie in den folgenden Beispielen gezeigt wird, reagiert das erfindungsgemäße Verfahren (bei HIV Patienten) in der Regel schon mehrere Jahre vor der klinischen Diagnose eines Kaposi Sarkoms positiv. Da zur Zeit verschiedene Antiherpesmittel als mögliche KS Therapeutika in klinischer Erprobung sind, könnten diese oder andere antivirale Substanzen (sofern sie sich in der Behandlung von KS als wirksam erweisen) möglicherweise präventiv eingesetzt werden, sobald eine Infektion mit HHV8 sicher mit dem erfindungsgemäßen Verfahren/Kit nachgewiesen wird.

Die Beispiele erläutern die Erfindung.

### Beipiel 1: ELISA Test

Für den KS Peptid ELISA werden N-terminal biotinylierte, 14 Aminosäuren lange Peptide (10 Aminosäuren des jeweiligen Antigens, Ser-Gly-Ser-Gly Tetrapeptid als Spacer) eingesetzt. Die Stammlösung der Peptide (1 µmol/ml in 100% DMSO) wird zur Beschichtung der Streptavidin-Mikrotiterplatten (Combiplate 8, Life Sciences oder MC, MicroCoat) 1:400 in PBS/5% Tween 20 verdünnt; hiervon werden jeweils 100 µl pro Well der MTP eingesetzt (250 pmol, d.h. in 10fachem Überschuß gegenüber der verfügbaren Menge an gebundenem Streptavidin) und für 30 Minuten bei Raumtemperatur inkubiert. Die Platten werden darauf 2x mit PBS/0,2% Tween 20 gewaschen und dann 1 Stunde bei Raumtemperatur mit PBS/5% Tween 20 geblockt. Vor der Inkubation mit dem Testmaterial (z.B. 1:100 verdünntes Patientenserum) für 1 Stunde bei Raumtemperatur werden die Platten 3x mit PBS/0,2% Tween 20 gewaschen.
Nach der Inkubation mit dem Testmaterial werden die Platten 5x gewaschen und danach mit einer 1:10.000 Verdünnung eines Peroxidase-konjugierten anti-Human IgG oder IgM Antikörpers für 1 Stunde bei Raumtemperatur inkubiert. Anschließend folgen 5 Waschgänge, die beiden letzten mit PBS ohne Tween.
Zum Nachweis gebundenen Konjugats werden 200 µl einer 1mg/ml ABTS Lösung in Peroxidase Arbeitspuffer (Boehringer Mannheim) pro Vertiefung der Mikrotiterplatte hinzugegeben und die Platten 30 Minuten bei Raumtemperatur inkubiert. Die Messung der Absorption erfolgt bei 405 nm gegen eine Referenzwellenlänge von 492 nm. Die Probe wird als positiv gewertet, wenn der Meßwert >200 mO.D. über dem Mittelwert der Kontrolle (ohne Peptidgemisch) liegt (200 mO.D. entsprechen mind. 4 Standardabweichungen des Mittelwerts).

### Beipiel 2: KS Serumpanel

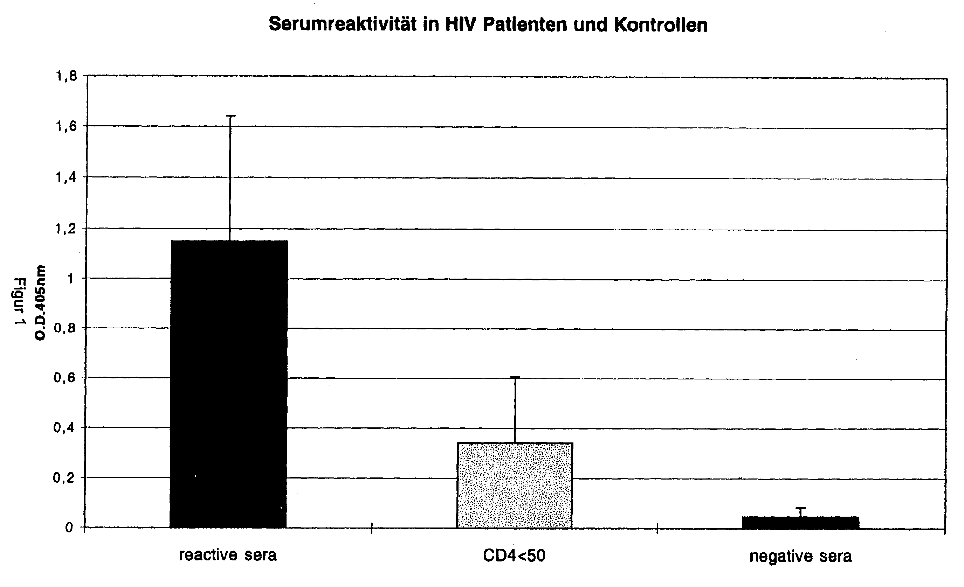

Zur serologischen Bestätigung der Wirksamkeit wurde zunächst ein Panel von 380 Einzelseren von 185 Patienten mit einem Kaposi Sarkom untersucht. Von den 256 Seren, die nach der KS Diagnose entnommen wurden, reagierten 246 positiv mit dem bevorzugten Gemisch 1 (Peptide Nr. 1, 3, 4 und 8). Dies entspricht einer Sensitivität von 96,1%. Da in diesem Panel etliche Seren von HIV Patienten vertreten sind, bei denen das Immunsystem durch die fortschreitende HIV Infektion bereits stark geschädigt war (dies schränkt auch die Aktivierung der Antikörper produzierenden Zellen ein) ist die Sensitivität des Tests mit anderen Panels vermutlich noch höher.
79 von 96 Seren von Patienten mit stark eingeschränkter Funktion des Immunsystems (CD4-Werte < 50) reagierten positiv mit dem beschriebenen Peptidgemisch; der Mittelwert der Absorption bei 405 nm betrug 0,342, die Standardabweichung 0,264. Für die negativen Seren wurde ein Mittelwert von 0,046 und eine Standardabweichung von 0,038 ermittelt. 120 Seren zeigten eine starke Immunreaktivität (O.D.₄₀₅ₙₘ > 0,6). Für diese Seren liegt der Mittelwert der Absorption bei 405 nm von 1,15 ± 0,49 O.D.(Figur 1).

### Beipiel 3: Assay-Standardisierung

Zur Standardisierung des Assays wurden verschiedene Platten, Blockierungsmethoden und-Verdünnungsmittel getestet. Eine gleichbleibende Qualität der Plattenbeschichtung und optimierte Reaktionsbedingungen bestimmen sowohl die Sensitivität als auch die Reproduzierbarkeit des Assays maßgeblich mit. Der beschriebene KS ELISA weist mit dem bevorzugten Peptidgemisch sehr geringe Intra- und Interassay Varianzen auf. Der mittlere Varianzkoeffizient der 79 Doppelbestimmungen niedrigreaktiver Seren ist 4,39%. Bei den Patienten, von denen Verlaufsseren vorlagen, wurden keine Abweichungen in den Testergebnissen sukzessiver Serumproben festgestellt, nachdem eine Probe im Test einmal positiv war. Die Blankwerte (Inkubation nur mit Serum ohne Peptide) sind gut reproduzierbar (Mittelwert aus 30 Platten: 128 ± 45 milli O.D.)

### Beipiel 4: Kontrollpanels

Verschiedene Kontrollpanels sowie Kontrollpeptide wurden eingesetzt, um die Spezifität des HHV8 Antikörper ELISAs zu testen. Mit einem EBV Kontrollpeptid (siehe Tabelle 2) wurde untersucht, in wie vielen Fällen vergleichbare Antikörpertiter gegen HHV8 bzw. EBV (ein damit verwandtes Herpesvirus) nachgewiesen werden konnten. In 56 von 64 Fällen waren die beobachteten Antikörpertiter um mindestens 30% divergent; die EBV Durchseuchungsrate lag bei den HIV⁺ Patienten bei 86%, bei den Kontrollseren bei 72%. 28 von 99 Seren zeigten sowohl gegen HHV8 als auch EBV eine hohe Immunreaktivität (0.D.₄₀₅nm > 0,6), 39 von 99 Seren hatten hohe anti-HHV8 und niedrige anti-EBV Titer, 7 von 99 niedrige anti-HHV8 und hohe anti-EBV Titer, bei 25 der 99 Seren waren sowohl die anti-HHV8 als auch die anti-EBV Immunreaktivität kleiner als 0,6. Aus diesen Daten läßt sich folgern, daß keine signifikante Kreuzreaktion des Assays mit EBV-Antigenen vorliegt.

### Beipiel 5: Sensitivität

Ein hochreaktives Einzelserum wurde in Verdünnungen von 1:50 bis 1:12800 getestet. In dem kolorimetrischen Assay liefert selbst die höchste Serumverdünnung eine O.D.₄₀₅ₙₘ> 1. Die mögliche Bandbreite des Assays ist hier durch das relativ schmale nutzbare Fenster des kolorimetrischen Detektionssystems limitiert. Durch den Einsatz verbesserter Nachweisverfahren (z.B. Chemilumineszenz) dürften lineare Bestimmungen der Antikörpertiter über einen Bereich von mehr als 3 Zehnerlogarithmen möglich sein. So könnten sowohl hochtitrige Seren quantifiziert werden als auch geringe Antikörpertiter sicher erkannt werden.

### Beipiel 6: Prognostischer Wert

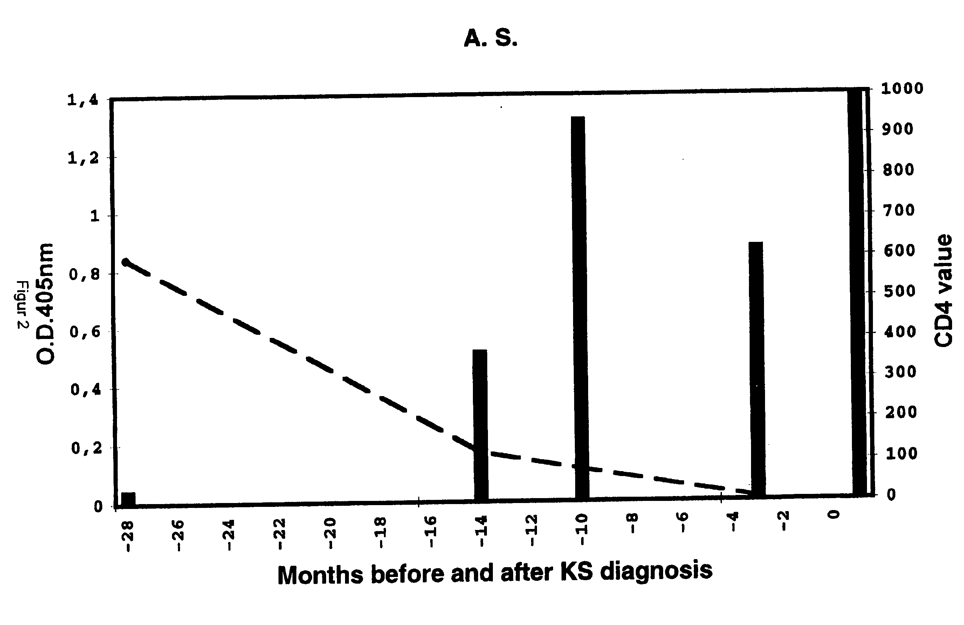

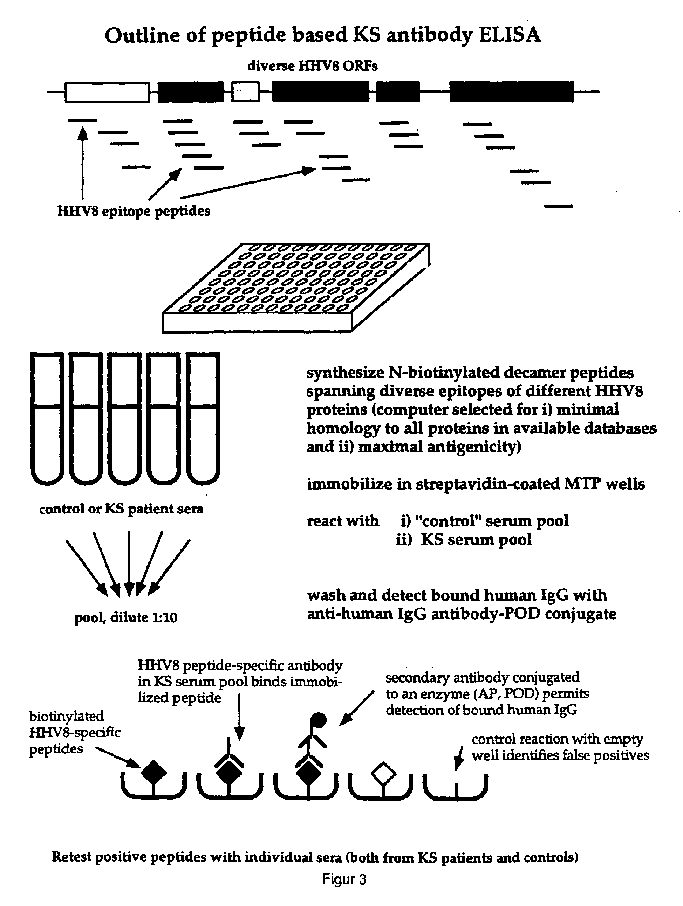

Von 20 KS Patienten, welche über einen längeren Zeitraum in Behandlung waren, wurden sukzessive Serumproben auf Immunreaktivität HHV8-spezifischer Antikörper untersucht. Der Zeitpunkt der jeweils ersten positiven Serumprobe lag im Schnitt 20,4±14,6 Monate vor der Erstdiagnose eines Kaposi Sarkoms. Da in den meisten Fällen die erste verfügbare Serumprobe bereits positiv getestet wurde, ist die tatsächliche "Inkubationszeit" wahrscheinlich länger. Der ELISA könnte somit Hinweise auf ein bestehendes Risiko für die Entwicklung eines Kaposi Sarkoms liefern. Die Verlaufskurve eines dieser Langzeitpatienten ist in Figur 2 dargestellt.

### Beipiel 7: Ausschluß falschpositiver Test-Ergebnisse

Von insgesamt 589 getesteten KS und Kontrollseren reagierten 8 (1.36%) mit den leeren, nur mit Streptavidin allein beschichteten Kavitäten der Testmikrotiterplatten. Alle dieser Seren waren nach Vorinkubation auf Platten ohne Peptid negativ im HHV8 ELISA. Diese falsch-positiven Werte können durch die Verwendung probenspezifischer Blankwerte daher sicher ausgeschlossen werden. Solche serenspezifischen Blankwerte werden in keinem der bisher beschriebenen Testsysteme erfaßt, weswegen die aus unspezifisch reaktiven Seren resultierenden falschpositiven Ergebnisse nicht erfaßt werden können. Diese zusätzliche Kontrolle verhindert den unnötigen Ausschluß von Blutpräparaten und ist daher speziell für die Anwender in Blutbanken auch vom wirtschaftlichen Gesichtspunkt her interessant. Wie erste Tests ergeben haben, scheint in Serumpanels aus südeuropäischen bzw. afrikanischen Ländern der Anteil unspezifisch reaktiver Seren zudem weit höher zu sein.

### Beipiel 8: Vergleich des Antikörpertests mit DNA Nachweisverfahren

Von 100 Probanden wurden sowohl Serumproben als auch Präparationen genomischer DNA aus unfraktionierten peripheren Blutzellen hergestellt. Die Seren wurden in dem HHV8 Peptid-ELISA analysiert, die HHV8 DNAs mit einer Polymerasekettenreaktion (PCR) mit einer Nachweisgrenze von 5-10 Kopien/Reaktion (=1000-2000 Kopien/ml Blut) bestimmt. 5% der Probanden testeten positiv, obwohl der kontrollierte PCR-Nachweis negativ war.

### Literatur

1. Beral, V., Peterman, T.A., Berkelman, R.L. & Jaffe, H.W. Kaposi's sarcoma among persons with AIDS: a sexually transmitted infection? Lancet 335, 123-128 (1990).
2. Kaposi, M. Idiopathisches multiples Pigmentsarkom der Haut. Arch. Dermatol. Syphil. 4, 742-749 (1872).
3. Archibald, C.P. et al. Evidence for a sexually transmitted cofactor for AIDS-related Kaposi's sarcoma in a cohort of homosexual men. Epidemiology 3, 203-209 (1992).
4. Chang, Y. et al. Identification of herpesvirus-like DNA sequences in AIDS-associated Kaposi's sarcoma. Science 266, 1865-1869 (1994).
5. Russo, J.J. et al. Nucleotide sequence of the Kaposi sarcoma associated herpesvirus (HHV8). Proc. Natl. Acad. Sci. (U.S.A.) 93, 14862-14867 (1996).
6. Dupin, N. et al. Herpesvirus-like DNA sequences in patients with Mediterranean Kaposi's sarcoma. Lancet 345, 761-762 (1995).
7. Huang, Y.Q. et al. Human herpesvirus-like nucleic acid in various forms of Kaposi's sarcoma. Lancet 345, 759-761 (1995).
8. Luppi, M. et al. Frequency and distribution of herpesvirus-like DNA sequences (KSHV) in different stages of classic Kaposi's sarcoma and in normal tissues from an Italian population. Int J Cancer 66, 427-431 (1996).
9. Moore, P.S. & Chang, Y. Detection of herpesvirus-like DNA sequences in Kaposi's sarcoma in patients with and without HIV infection. N Engl J Med 332, 1181-1185 (1995).
10. Moore, P.S. et al. Kaposi's sarcoma-associated herpesvirus infection prior to onset of Kaposi's sarcoma. Aids 10, 175-180 (1996).
11. O'Neill, E. et al. Herpes virus-like sequences are specifically found in Kaposi sarcoma lesions. J Clin Pathol 49, 306-308 (1996).
12. Whitby, D. et al. Detection of Kaposi sarcoma associated herpesvirus in peripheral blood of HIV-infected individuals and progression to Kaposi's sarcoma. Lancet 346, 799-802 (1995).
13. Soulier, J. et al. Kaposi's sarcoma-associated herpesvirus-like DNA sequences in multicentric Castleman's disease. Blood 86, 1276-1280 (1995).
14. Lin, J.C. et al. Is Kaposi's-sarcoma-associated herpesvirus detectable in semen of HIV-infected homosexual men? Lancet 346, 1601-1602 (1995).
15. Said, J.W., Tasaka, T., Devos, S. & Koeffler, H.P. Kaposis sarcoma associated herpesvirus human herpesvivus type 8 encephalitis in HIV positive and negative individuals. Aids 11, 1119-1122 (1997).
16. Rettig, M.B. et al. Kaposi's sarcoma-associated herpesvirus infection of bone marrow dendritic cells from multiple myeloma patients. Science 276, 1851-1854 (1997).
17. Lunardi-Iskandar, Y. et al. Tumorigenesis and metastasis of neoplastic Kaposi's sarcoma cell line in immunodeficient mice blocked by a human pregnancy hormone. Nature 375, 64-68 (1995).
18. Mori, S., Murakami, M.K., Jewett, A., Nakamura, S. & Bonavida, B. Resistance of AIDS-associated Kaposi's sarcoma cells to Fas-mediated apoptosis. Cancer Res 56, 1874-1879 (1996).
19. Cheng, E.H.Y. et al. A bei 2 homolog encoded by Kaposi Sarcoma associated virus, human herpesvirus 8, inhibits apoptosis but does not heterodimerize with bax or bak. Proc. Natl. Acad. Sci. (U.S.A.) 94, 690-694 (1997).
20. Molden, J., Chang, Y., You, Y., Moore, P.S. & Goldsmith, M.A. A Kaposi's sarcoma-associated herpesvirus-encoded cytokine homolog (vIL-6) activates signaling through the shared gp130 receptor subunit. J Biol Chem 272, 19625-19631 (1997).
21. Cesarman, E. et al. Kaposi's sarcoma-associated herpesvirus contains G proteincoupled receptor and cyclin D homologs which are expressed in Kaposi's sarcoma and malignant lymphoma. J Virol 70, 8218-8223 (1996).
22. Li, M. et al. Kaposi's sarcoma-associated herpesvirus encodes a functional cyclin. J Virol 71, 1984-1991 (1997).
23. Tasaka, T. et al. Is Kaposi's sarcoma-associated herpesvirus ubiquitous in urogenital and prostate tissues? Blood 89, 1686-1689 (1997).
24. Gao, S.J. et al. KSHV antibodies among Americans, Italians and Ugandans with and without Kaposi's sarcoma. Nat Med 2, 925-928 (1996).
25. Cesarman, E., Chang, Y., Moore, P.S., Said, J.W. & Knowles, D.M. Kaposi's sarcoma-associated herpesvirus-like DNA sequences in AIDS-related body-cavitybased lymphomas. N Engl J Med 332, 1186-1191 (1995).
26. Lock, M.J., Griffiths, P.D. & Emery, V.C. Development of a quantitative competitive polymerase chain reaction for human herpesvirus 8. J Virol Methods 64, 19-26 (1997).
27. Cathomas, G. et al. Detection of herpesvirus-like DNA by nested PCR on archival skin biopsy specimens of various forms of Kaposi sarcoma. J Clin Pathol 49, 631-633 (1996).
28. Boshoff, C. et al. Kaposi's sarcoma-associated herpesvirus infects endothelial and spindle cells. Nat Med 1, 1274-1278 (1995).
29. Rainbow, L. et al. The 222- to 234-kilodalton latent nuclear protein (LNA) of Kaposi's sarcoma-associated herpesvirus (human herpesvirus 8) is encoded by ORF73 and is a component of the latency-associated nuclear antigen. J Virol 71, 5915-5921 (1997).
30. Kedes, D.H., Ganem, D., Ameli, N., Bacchetti, P. & Greenblatt, R. The prevalence of serum antibody to human herpesvirus 8 (Kaposi sarcoma-associated herpesvirus) among HIV-seropositive and high-risk HIV-seronegative women. Jama 277, 478-481 (1997).
31. Davis, D.A. et al. Detection of serum antibodies to a Kaposi's sarcoma-associated herpesvirus-specific peptide. J Infect Dis 175, 1071-1079 (1997).
32. Simpson, G.R. et al. Prevalence of Kaposi's sarcoma associated herpesvirus infection measured by antibodies to recombinant capsid protein and latent immunofluorescence antigen. Lancet 348, 1133-1138 (1996).
33. Andre, S. et al. Detection of antibodies against viral capsid proteins of human herpesvirus 8 in AIDS-associated Kaposi's sarcoma. J. Mol. Med. 75, 145-152 (1997).
34. Miller, G. et al. Antibodies to butyrate-inducible antigens of Kaposi's sarcoma-associated herpesvirus in patients with HIV-1 infection. N Engl J Med 334, 1292-1297 (1996).

**TABELLE 1**

| Mit KS Seren reaktive Peptide. Die Positionsangaben beziehen sich auf die publizierte Sequenz des HHV8 Genoms von Russo et al. (5) (Genbank accession No. U75698). | | | | |
|---|---|---|---|---|
| **Nr.** | **Seq ID** | **Position** | **Sequenz** | **ORF** |
| **1** | KS20A | 35573-35544 | M Y E V F T D F P V | tegument |
| **2** | KS29bG | 50000-49971 | D P A Y T N N T E A | packaging protein |
| **3** | KS29bP | 49394-49365 | R H M Y K P I S P Q | packaging protein |
| **4** | KS65A | 112443-112414 | M S N F K V R D P V | capsid |
| **5** | KS65X | 111960-111931 | A R K P P S G K K K | capsid |
| **6** | KS73J | 124726-124697 | Q E E Q E L E E V E | LANA |
| **7** | KSK12D | 118023-117994 | A I P P L V C L L A | kaposin |
| **8** | KS8.1A | 75999-76034 | P T Y R S H L G F W Q E | glycoprotein |

**TABELLE 2**

| Sequenzen der verwendeten Kontroll-Peptide. Die Positionsangaben beziehen sich auf HIV-1 HXB2 (Genbank accession no. M38432), Epstein-Barr Virus Stamm B95-8 (V01555) sowie Poliovirus Typ Sabin 1 (V01150). Die jeweils ersten 4 Aminosäuren in kursiven Lettern (SGSG) wurden als Spacer eingefügt. | | | | |
|---|---|---|---|---|
| **Nr.** | **Seq ID** | **Position** | **Sequenz** | **ORF** |
| **1** | HIV B | 8007-8036 | SGSGIWGCSGKLIC | TM(gp41) |
| **2** | EBV A | 100695-100724 | SGSGOEPPAPQAPI | EBNA 6 |
| **3** | polio B | 2603-2632 | S G S G P A L T A V E T G A | VP 1 capsid |

## Patentansprüche

1. Verfahren zum Nachweis von anti-HHV8 Antikörpern, das folgende Schritte umfasst:
(a) Inkontaktbringen einer biologischen Probe mit mindestens einem Peptid das ein lineares Epitop darstellt und eine Länge von höchstens 15 Aminosäuren besitzt und **dadurch gekennzeichnet ist, dass** das Peptid
(i) eine der in SEQ ID NR: 1 bis 4, 6 und 8 gekennzeichneten Aminosäuresequenzen umfasst; oder
(ii) aus einer der in SEQ ID NR: 1 bis 4, 6 und 8 gekennzeichneten Aminosäuresequenzen besteht;
wobei das Peptid kein natürlich vorkommendes HHV8-Protein ist, unter Bedingungen, die die Bindung von Antikörpern erlauben; und
(b) Nachweis der in Schritte (a) gebundenen Antikörper.

2. Verfahren nach Anspruch 1, wobei das Peptid chemisch synthetisiert ist.

3. Verfahren nach einem der Ansprüche 1 oder 2, wobei ein Gemisch der Peptide, die die in SEQ ID NR.: 1, 3, 4 und 8 gekennzeichneten Aminosäuresequenzen umfassen, verwendet wird.

4. Verfahren nach Anspruch 3, wobei das Gemisch die Peptide in einem molaren Verhältnis von 1:1:1:1 umfasst.

5. Verfahren nach einem der Ansprüche 1 bis 4, das ein Enzym-gebundener Immunoabsorptionsassay (ELISA), ein Enzymimmunodotassay, ein Immunobead-Assay, ein passiver Hämagglutinations-Assay (PHA) oder ein Peptid-Antikörper-Peptid Sandwich-Assay ist.

6. Verfahren nach einem der Ansprüche 1 bis 5, wobei die biologische Probe Blut, Serum, Gewebeextrakt, Gewebeflüssigkeit, Zellkulturüberstand, oder Zellysat ist.

7. Verfahren nach einem der Ansprüche 1 bis 6, das den weiteren Schritt umfasst:
(c) Nachweis von unspezifischen Bindungen, die von in der biologischen Probe zusätzlich vorhandenen Antikörpern in Schritt (a) verursacht werden.

8. Kit, enthaltend
(a) mindestens ein Peptid wie in Anspruch 1 definiert, wobei die Komponenten getrennt voneinander oder in Form eines Gemisches enthalten sind; und gegebenenfalls
(b) eine feste Phase, an die mindestens ein Peptid nach Anspruch 1 gebunden werden kann;
(c) ein Probenverdonnungsmittel; und/oder
(d) eine Negativkontrolle; und/oder
(e) eine Positivkontrolle; und/oder
(f) ein Reportermotekül.

9. Kit nach Anspruch 8, der ein Gemisch der Peptide, die die in SEQ ID NR.: 1, 3, 4 und 8 gekennzeichneten Aminosäuresequenzen umfassen oder darstellen, enthält.

10. Verwendung mindestens eines Peptids wie in Anspruch 1 definiert zur Herstellung von Antikörpern.

11. Verwendung mindestens eines Peptids wie in Anspruch 1 definiert zum Nachweis von anti-HHV8 Antikörpern.

12. Peptid wie in Anspruch 1 definiert zur Verwendung in der Behandlung von HHV8-assoziierten Erkrankungen, wobei die HHV8-assoziierten Erkrankungen ausgewählt sind aus Kaposi-Sarkom, Castleman's Disease, Primary Effusion Lymphoma, interstitieller Pneumonie, Enzephalitis und multiplen Myelomen.

## Claims

1. A method for the detection of anti-HHV8 antibodies, comprising the following steps:
(a) contacting a biological sample with at least one peptide constituting a linear epitope and having a length of at the most fifteen amino acids **characterised in that** said peptide
(i) comprises one of the amino acid sequences presented in SEQ ID NOs.: 1 to 4, 6 and 8; or
(ii) consists of one of the amino acid sequences presented in SEQ ID NOs.: 1 to 4, 6 and 8;
wherein the peptide is not a naturally occurring HHV-8 protein, under conditions which allow the binding of antibodies; and
(b) detection of the antibodies bound in step (a).

2. The method according to claim 1, where the peptide is chemically synthesized.

3. The method according to claim 1 or 2, wherein a mixture of the peptides comprising the amino acid sequences presented in SEQ ID NOs.: 1, 3, 4 and 8 is used.

4. The method according to claim 3, wherein the mixture comprises the peptides in a molar ratio of 1:1:1:1.

5. The method according to any one of claims 1 to 4, which is an enzyme-linked immunosorbent assay (ELISA), an enzyme immunodot assay, an immunobead assay, a passive hemagglutination assay (PHA) or a peptide-antibody-peptide sandwich assay.

6. The method according to any one of claims 1 to 5, wherein the biological sample is blood, serum, tissue extract, tissue fluid, cell culture supernatant, or a cell lysate.

7. The method according to any one of claims 1 to 6, comprising the additional step of:
(c) detection of unspecific binding reactions caused by other antibodies in step (a) that are present in the biological sample.

8. A kit comprising:
(a) at least one peptide as defined in claim 1, whereby the components are separated from each other or in form of a mixture; and optionally
(b) a solid phase, to which at least one peptide according to claim 1 may be bound;
(c) a sample diluent; and/or
(d) a negative control; and/or
(e) a positive control; and/or
(f) a reporter molecule.

9. A kit according to claim 8, comprising a mixture of peptides, which represent or comprise the amino acid sequences **characterized in** SEQ ID NOs.: 1, 3, 4 and 8.

10. Use of at least one peptide as defined in claim 1 for the production of antibodies.

11. Use of at least one peptide as defined in claim 1 for the detection of anti-HHV8 antibodies.

12. A peptide as defined in claim 1 for use in the treatment of HHV8-associated diseases, wherein the HHV8-associated diseases are selected from Kaposi's sarcoma, Castleman's disease, primary effusion lymphoma, interstitial pneumonitis, encephalitis and multiple myeloma.

## Revendications

1. Procédé de détection d'anticorps anti-HHV8, comprenant les étapes suivantes :
(a) mise en contact d'un échantillon biologique avec au moins un peptide qui constitue un épitope linéaire et possède une longueur d'au maximum 15 acides aminés, et est **caractérisé en ce que** le peptide
(i) comprend l'une des séquences d'acides aminés caractérisées dans les SEQ ID N°1 à 4, 6 et 8 ; ou
(ii) est constitué de l'une des séquences d'acides aminés caractérisées dans les SEQ ID N°1 à 4, 6 et 8 ;
le peptide n'étant pas une protéine de HHV8 naturelle, dans des conditions permettant la liaison des anticorps ; et
(b) détection des anticorps liés à l'étape (a).

2. Procédé selon la revendication 1, dans lequel le peptide est synthétisé chimiquement.

3. Procédé selon l'une des revendications 1 ou 2, dans lequel on utilise un mélange de peptides qui comprennent les séquences d'acides aminés caractérisées dans les SEQ ID N°1, 3, 4 et 8.

4. Procédé selon la revendication 3, dans lequel le mélange comprend les peptides en un rapport molaire de 1/1/1/1.

5. Procédé selon l'une des revendications 1 à 4, qui est un test d'immunoabsorption enzymatique (ELISA), un test d'immuno-empreinte enzymatique, un test aux immunobilles, un test d'hémagglutination passive (PHA) ou une analyse Sandwich peptide - anticorps - peptide.

6. Procédé selon l'une des revendications 1 à 5, dans lequel l'échantillon biologique est du sang, du sérum, un extrait de tissu, du liquide tissulaire, un surnageant de culture cellulaire ou un lysat cellulaire.

7. Procédé selon l'une des revendications 1 à 6, qui comprend en outre l'étape de:
(c) détection des liaisons non spécifiques qui sont causées à l'étape (a) par des anticorps supplémentaires présents dans l'échantillon biologique.

8. Kit comprenant:
(a) au moins un peptide tel que défini dans la revendication 1, dans lequel les composants sont présents séparément les uns des autres ou sous la forme d'un mélange ; et le cas échéant
(b) une phase solide, à laquelle peut être lié au moins un peptide selon la revendication 1 ;
(c) un moyen de dilution des échantillons ; et/ou
(d) un témoin négatif ; et/ou
(e) un témoin positif ; et/ou
(f) une molécule rapporteuse.

9. Kit selon la revendication 8, qui comprend un mélange des peptides qui comprennent ou sont constitués par les séquences d'acides aminés caractérisées dans les SEQ ID N°1, 3, 4 et 8.

10. Utilisation d'au moins un peptide tel que défini dans la revendication 1 pour la préparation d'anticorps.

11. Utilisation d'au moins un peptide tel que défini dans la revendication 1 pour la détection d'anticorps anti-HHV8.

12. Peptide tel que défini dans la revendication 1 pour son utilisation dans le traitement de maladies associées au HHV8, dans lequel les maladies associées au HHV8 sont choisies parmi le sarcome de Kaposi, la maladie de Castleman, le lymphome primaire d'effusion, la pneumonie interstitielle, l'encéphalite et les myélomes multiples.
